# EUROPÄISCHE PATENTSCHRIFT

(11) **EP 0 737 454 B1**
(45) Veröffentlichungstag und Bekanntmachung des Hinweises auf die Patenterteilung: **24.11.1999**
(21) Anmeldenummer: 96105593.6
(22) Anmeldetag: 09.04.1996
(51) Int. Cl.: A61F 2/30

(54) **Endoprothese, insbesondere für das Sternum-Clavicular-Gelenk**
Endoprosthesis, in particular for the sterno-clavicular joint
Endoprothèse, en particulier pour l'articulation sterno-claviculaire

(30) Priorität: 13.04.1995 DE 29506419 U
(43) Veröffentlichungstag der Anmeldung: 16.10.1996
(73) Patentinhaber: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 214 773
- EP-A- 0 502 815
- FR-A- 2 558 721
- GB-A- 2 045 085
- US-A- 3 815 157
- US-A- 4 106 128
- US-A- 4 892 545

## Beschreibung

Das Schlüsselbein (Claviculum) und das Brustbein (Sternum) sind miteinander über ein Knorpelgelenk verbunden, dessen Beschädigung mit Beschwerden verbunden ist. Man könnte daran denken, das Gelenk durch eine Prothese zu ersetzen, Jedoch sind bekannte Prothesentypen dafür nicht geeignet, weil diese in der Regel bei der Implantation eine Distraktion der durch die Prothese zu verbindenden Knochen verlangen. Das Schlüsselbein und das Brustbein sind jedoch nur schwer distrahierbar.

Der Erfindung liegt daher die Aufgabe zugrunde, eine Endoprothese zum Ersatz eines Gelenks zwischen nicht oder nur schwer voneinander distrahierbaren Knochen, insbesondere für das Sternum-Clavicular-Gelenk zu schaffen.

Die erfindungsgemäße Lösung besteht in den Merkmalen des Anspruchs 1 und vorzugsweise demjenigen der Unteransprüche.

Die Erfindung wählt als Prothesentyp denjenigen eines Universalgelenks, d.h. eines Gelenks, das in sämtlichen Richtungen wie ein Kugelgelenk schwenkbar ist, wenn auch in einem begrenzten Winkelbereich. Diese Möglichkeit wird dadurch geschaffen, daß eine Komponente der Prothese mit einem Gelenkköpfchen versehen ist, das von einem an der anderen Komponente angeordneten Umfassungsteil schwenkbar eingeschlossen ist. Diese Bauweise ist bei Prothesen an sich bekannt (US-A 4 106 128; FR-A 2 558 721; GB-A 2 045 085; US-A 3 815 157; EP-A 214 773). Sie bringt es mit sich, daß - nachdem die Prothesenkomponenten je für sich in die zugehörigen Knochen eingesetzt worden sind - die Knochen distrahiert werden müssen, damit das Gelenkköpfchen in den Umfassungsteil eingesetzt werden kann. Da das Schlüsselbein und das Brustbein aber nur schwer distrahierbar sind, sieht die Erfindung vor, daß das Gelenkköpfchen durch eine von seitwärts her kommende Bewegung in den Umfassungsteil eingefügt wird. Dies wird dadurch ermöglicht, daß der Umfassungsteil eine seitliche Montageöffnung aufweist.

Die beiden Prothesenkomponenten weisen jeweils einen Befestigungsteil auf, mittels dessen sie am zugehörigen Knochen befestigt werden. Dabei kann es sich um schaftartige Teile handeln, wie sie an sich bekannt sind. Der Umfassungsteil umfaßt das Köpfchen über mindestens 180°, zweckmäßigerweise mehr als 180°, um einen sicheren Schwenksitz für das Köpfchen zu bilden. Der Umfassungsteil hat eine Halsöffnung zur Durchführung des Halses, der das Köpfchen mit dem zugehörigen Befestigungsteil verbindet. Wenn der Befestigungsteil schaftförmig ist, kann der Halsteil der dem Köpfchen nächste Bereich des Schaftes sein, wobei der Halsteil praktisch ein Teil des Schaftes ist.

Bei der Operation geht man so vor, daß zunächst die beiden Prothesenteile mit den zugehörigen Knochen verbunden werden, während sie im Vergleich mit ihrer natürlichen Verbindungslinie gegeneinander versetzt sind. Danach werden sie miteinander verbunden, indem das Köpfchen durch die seitliche Montageöffnung in den Umfassungsteil eingeführt wird. Zweckmäßigerweise ist ein Schließteil vorgesehen, mittels dessen die Montageöffnung anschließend geschlossen werden kann, so daß das Köpfchen aus dem Umfassungsteil nicht mehr entweichen kann.

Der Umfassungsteil kann unterschiedliche Gestalt annehmen. Zweckmäßigerweise wird er von einem mit dem zugehörigen Befestigungsteil verbundenen, topfförmigen Teil gebildet, dessen Seitenwand zur Bildung der Montageöffnung offen ist.

Dabei bildet der Schließteil zweckmäßigerweise einen mit der Seitenwand des topfförmigen Teils verbindbaren Ring, der mittels eines Gewindes, eines Bajonettverchlusses, eines Schnappverschlusses oder dergleichen mit dem topfförmigen Teil verbunden werden kann.

Um Metall-Metall-Kontakt zwischen dem Gelenkköpfchen und dem Umfassungsteil zu vermeiden, ist zweckmäßigerweise zwischen diesen Teilen ein Gleitmantel aus gleitgünstigem Werkstoff wie Polyethylen angeordnet. Dieser Gleitmantel ist zweckmäßigerweise seitlich ringsum geschlossen ausgebildet, wobei die Weite der Montageöffnung des Umfassungsteils mindestens dem Außendurchmesser des Gleitmantels entspricht. Dies hat den Vorteil, daß der Gleitmantel vor dem Zusammenführen der Prothesenkomponenten auf dem Köpfchen montiert werden kann und das Köpfchen zusammen mit dem Gleitmantel in den Umfassungsteil eingeschoben werden kann.

Zweckmäßigerweise weist der Gleitmantel eine Öffnungsverengung auf, die von dem Gelenkköpfchen mit elastischer Dehnung des Gleitmantels passierbar ist. Dabei wird die Außenseite des Gleitmantels zweckmäßigerweise derart von dem Umfassungsteil abgestützt, daß der Gleitmantel sich nicht dehnen kann und somit das Gelenkköpfchen dem Gleitmantel im montierten Zustand nicht mehr entweichen kann. Es können auf diese Weise auch Zugkräfte von der Prothese übertragen werden. Der Durchmesser der Halsöffnung in dem ringförmig ausgebildeten Schließteil darf dabei größer sein als der Außendurchmesser des Gelenkköpfchens, weil lediglich der Gleitmantel von dem Schließteil festgehalten werden muß.

Das Gelenkköpfchen ist zweckmäßigerweise sphärisch, um durch Gleitbewegung seiner Oberfläche gegenüber dem Gleitmantel die Gelenkbewegung zu ermöglichen. Statt dessen ist es aber auch möglich, das Gelenkköpfchen anders zu gestalten, beispielsweise als Flanschplatte am Ende des Halsteils, die von einem elastischen Bett innerhalb des Umfassungsteils umgeben ist, wobei die Gelenkbewegung durch Verformung des elastischen Betts ermöglicht wird. In diesem Fall kann das Gelenkköpfchen unrund sein und von einem das elastische Bett bildenden Mantel umgeben sein.

Die Erfindung wird im folgenden näher unter Bezugnahme auf die Zeichnung erläutert, die ein vorteilhaftes Ausführungsbeispiel veranschaulicht. Darin zeigen:
- Fig.1: eine Seitenansicht der Prothese in etwa natürlichem Maßstab,
- Fig.2: die Prothese während der Montage,
- Fig.3: einen Querschnitt gemäß Linie III-III der Fig.4 in größerem Maßstab und
- Fig.4 u. 5: einen Längsschnitt und eine Seitenansicht in größerem Maßstab.

Die dargestellte Prothese für ein Sternum-Clavicular-Gelenk besteht aus der Sternum-Komponente 1, die mit dem Brustbein zu verbinden ist, und der Clavicular-Komponente 2, die mit dem Schlüsselbein zu verbinden ist. Der Schaft 3 der Sternum-Komponente 1 bildet deren Befestigungsteil und ist fest verbunden mit einem Umfassungsteil 4, während der Schaft 5, der den Befestigungsteil des Clavicular-Prothesenteils bildet, mit einem sphärischen Gelenkköpfchen 6 verbunden ist, das von dem Umfassungsteil 4 aufgenommen ist. Die beiden Prothesenkomponenten sind gegeneinander um den Mittelpunkt des Gelenkköpfchens 6 bzw. des Umfassungsteils 4 in allen Richtungen schwenkbar und außerdem um die gemeinsame Längsachse drehbar. Sie können Druckkräfte und mäßige Zugkräfte in Längsrichtung übertragen. Der Begriff Längsrichtung bezieht sich dabei auf die durch die Längsachse 7 des Umfassungsteils 4 festgelegte Achse. Der Begriff seitlich bezieht sich auf Richtungen, die seitlich bzw. quer zu dieser Achse 7 liegen.

Die Befestigungsteile 3, 5 der beiden Prothesenkomponenten haben im Ausführungsbeispiel die Form von Schäften, wie dies dargestellt ist, die auch in geeigneter Weise gekrümmt sein können. Jedoch können die Befestigungsteile auch andere Gestalt annehmen.

Der Umfassungsteil 4 setzt sich aus einem topfförmigen Teil 8 und einem Schließring 9 zusammen. Der topfförmige Teil 8 besitzt einen Boden 11, der einstückig mit dem Schaft 3 verbunden ist, und eine Seitenwand 12. Der Schließring 9 hat einen zylindrischen Teil 13, der über ein Gewinde 14 mit der Wand 12 des topfförmigen Teils 8 verbindbar ist, und einen Flanschteil 15, der eine Öffnung 16 bildet. Diese wird als Halsöffnung bezeichnet, weil sie den Durchlaß darstellt für einen Hals 17, der zwischen dem Gelenkköpfchen 6 und dem Befestigungsteil 5 vorgesehen ist. Er liegt im Bereich der Halsöffnung 16 und hat einen geringeren Durchmesser als diese, um Schwenkbewegung der Prothesenkomponente 2 zu ermöglichen. Im vorliegenden Fall wird der Halsteil 17 von dem dem Gelenkköpfchen 6 naheliegenden Bereich des schaftförmigen Befestigungsteils 5 gebildet, ohne sich im Querschnitt von diesem zu unterscheiden.

Das Gelenkköpfchen 6 sitzt in einem Gleitmantel 18 aus Polyethylen, der töpfchenförmig gestaltet ist. Seine Außenform ist zylindrisch passend zum Innendurchmesser und zur inneren Länge des Umfassungsteils 8, 9. Seine Höhlung ist (im Längsschnitt gesehen) über mehr als 180° sphärisch gestaltet. Seine freie Öffnung 19 hat einen Innendurchmesser, der geringer ist als der Durchmesser des Gelenkköpfchens 6. Dank der Materialnachgiebigkeit des Gleitmantels 18 kann dieser auf das Gelenkköpfchen 6 aufgeschnappt werden, solange er noch nicht von dem Umfassungsteil 8 eingeschlossen sind. Wenn aber der Umfassungsteil den Gleitmantel 18 passend umgibt, hindert er diesen daran, sich elastisch zu dehnen, so daß das Gelenkköpfchen 6 auch beim Auftreten von in Längsrichtung wirkenden Zugkräften in dem Gleitmantel festgehalten wird. Der Innendurchmesser der Halsöffnung 16 des Schließrings 9 ist etwas geringer als der Außendurchmesser des Gleitmantels 18, so daß dieser im Umfassungsteil festgehalten wird. Jedoch kann die Halsöffnung 16 größer sein als der Außendurchmesser des Gelenkköpfchens 6.

Die Seitenwand 12 des topfförmigen Teils 8 des Umfassungsteils 4 hat eine seitliche Öffnung 20, die von den Kanten 21, 22, 23 begrenzt wird. Diese Öffnung 20 erstreckt sich nicht über die volle Länge der Seitenwand 12 bis zum Bodenteil 11, sondern nur über einen Teil derselben, so daß im Bereich der Öffnung 20 ein Wandteil 25 stehen bleibt. Daher ist der Gleitmantel 18 auch auf derjenigen Seite, auf der sich die Öffnung 20 befindet, rings von der Wand 12, 25 eingeschlossen und dadurch sicher gehalten, solange der Flansch 15 des Schließrings 9 ihn daran hindert, sich in Längsrichtung so weit zu bewegen, bis seine Unterkante die Öffnungskante 22 erreicht.

Der zylindrische Teil 13 des Schließrings 9 enthält eine Sicherungszunge 26, die durch zwei achsparallele Einschnitte 27 gebildet ist und im montierten Zustand über der Öffnung 20 positioniert wird und ein wenig in diese hineingebogen wird. Der Schließring kann sich dann im Öffnungssinne nur so weit drehen, bis die Sicherungszunge 26 die Kante 23 erreicht hat, und wird in dieser Lage festgehalten.

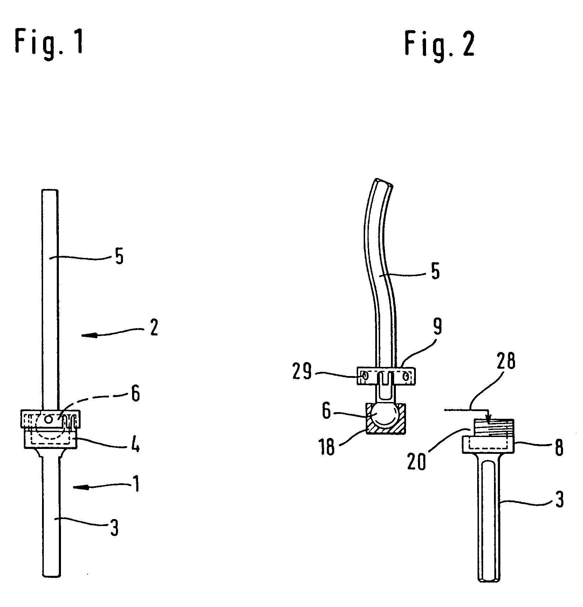

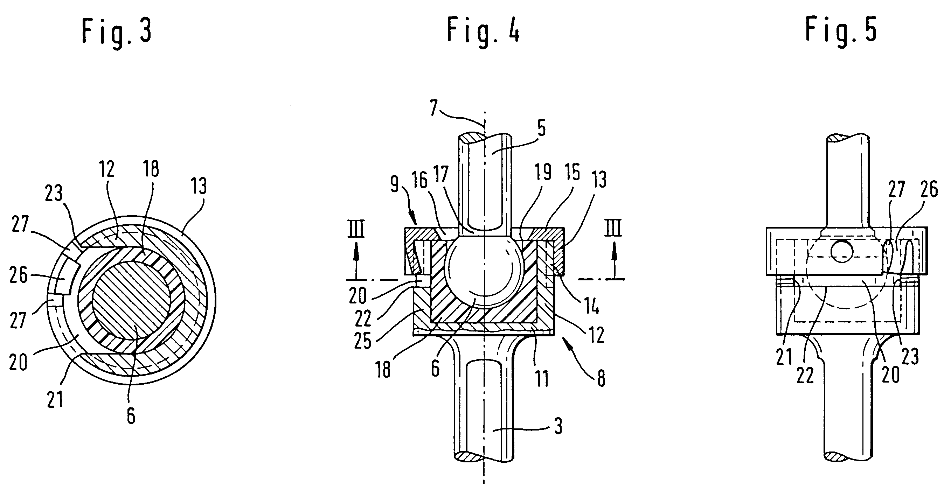

Beim Einsetzen der Prothese werden zunächst die Prothesenkomponenten 1, 2 mit dem Schlüsselbein bzw. dem Brustbein in bekannter Weise verbunden, wobei diese gegenüber der um die Achse 7 fluchtende Lage derart versetzt sind, wie dies beispielsweise in Fig. 2 gezeigt ist. Es wird dann der Schließring 9 über das Gelenkköpfchen 6 geschoben und der Gleitmantel 18 aufgeschnappt. Danach wird das Gelenkköpfchen 6 mit dem Gleitmantel 18 im Sinne des Pfeils 28 durch die Öffnung 20 hindurch in den topfförmigen Teil 8 hineingeschoben. Durch axiale Näherung der Prothesenteile gelangt der Gleitmantel in die hinter dem Wandteil 25 gebildete Vertiefung. Der Schließring 9 wird auf den topfförmigen Teil 8 aufgeschraubt. Dies ist möglich durch Angriff eines Werkzeugs an den über den Umfang verteilten Bohrungen 29 des Schließrings. Schließlich wird die Sicherungszunge 26 in die Sicherungslage gemäß Fig.5 gebogen.

Man erkennt, daß eine Distraktion der durch die Prothese zu verbindenden Knochen lediglich um die geringe Höhe des Wandteils 25 notwendig ist. Diese Höhe kann sich auf weniger als 2mm beschränken.

## Patentansprüche

1. Endoprothese für ein Gelenk zwischen zwei nicht oder nur schwer voneinander distrahierbaren Knochen, insbesondere für das Sternum-Clavicular-Gelenk, mit zwei Prothesenkomponenten (1,2), die je einen Befestigungsteil (3,5) zum Befestigen am zugehörigen Knochen aufweisen und ein Gelenk bilden, das aus einem mit dem ersten (5) der beiden Befestigungsteile über einen Halsteil (17) verbundenen Gelenkköpfchen (6) und einem mit dem zweiten Befestigungsteil (3) verbundenen, das Gelenkköpfchen (6) schwenkbeweglich aufnehmenden Umfassungsteil (4) besteht, der eine von dem mit ihm verbundenen Befestigungsteil (3) abgewendete Halsöffnung (16) für den Durchgang des Halsteils (17) aufweist, dadurch gekennzeichnet, daß der Umfassungsteil (4) eine in bezug auf die Halsöffnung (16) seitlich angeordnete Montageöffnung (20) zum Einführen des Köpfchens in den Umfassungsteil aufweist.

2. Endoprothese nach Anspruch 1, dadurch gekennzeichnet, daß der Umfassungsteil (4) zum Festhalten des Gelenkköpfchens (6) mit einem Schließteil (9) versehen ist.

3. Endoprothese nach Anspruch 1 oder 2, dadurch gekennzeichnet, daß der Umfassungsteil (4) einen mit dem zugehörigen Befestigungsteil (3) verbundenen topfförmigen Teil (8) aufweist, dessen Seitenwand (12) die Montageöffnung (20) enthält.

4. Endoprothese nach einem der Ansprüche 2 bis 3, dadurch gekennzeichnet, daß der Schließteil (9) ein mit der Wand (12) des topfförmigen Teils (8) über ein Gewinde (14) oder dergleichen zusammenwirkender Ring ist.

5. Endoprothese nach einem der Ansprüche 1 bis 4, dadurch gekennzeichnet, daß zwischen dem Gelenkköpfchen (6) und dem Umfassungsteil (4) ein Gleitmantel (18) aus gleitgünstigem Werkstoff wie Polyethylen angeordnet ist.

6. Endoprothese nach Anspruch 5, dadurch gekennzeichnet, daß der Gleitmantel seitlich ringsum geschlossen ausgebildet ist und die Weite der Montageöffnung (20) mindestens dem Außendurchmesser des Gleitmantels (18) entspricht.

7. Endoprothese nach Anspruch 5 oder 6, dadurch gekennzeichnet, daß der Gleitmantel (18) eine Öffnungsverengung (19) aufweist, die von dem Gelenkköpfchen (6) unter elastischer Dehnung des Gleitmantels passierbar ist, und daß der Gleitmantel (18) von dem Umfassungsteil (4) dehnungshindernd abgestützt ist.

8. Endoprothese nach einem der Ansprüche 1 bis 7, dadurch gekennzeichnet, daß der Durchmesser der von dem Schließteil (9) gebildeten Halsöffnung (16) größer als der des Gelenkköpfchens (6) aber kleiner als der öffnungsseitige Außendurchmesser des Gleitmantels (18) ist.

9. Endoprothese nach einem der Ansprüche 1 bis 8, dadurch gekennzeichnet, daß das Gelenkköpfchen sphärisch ist.

10. Endoprothese nach einem der Ansprüche 1 bis 8, dadurch gekennzeichnet, daß das Gelenkköpfchen (6) unrund und von einem elastischen Mantel umgeben ist.

## Claims

1. Endoprosthesis for a joint between two bones which cannot be distracted, or which can be distracted only with difficulty, in particular for the sternoclavicular joint, with two prosthesis components (1, 2) which each have a fastening part (3, 5) for fastening on the associated bone and which form a joint which consists of a joint head (6) which is connected to the first (5) of the two fastening parts via a neck part (17), and of an enclosure part (4) which is connected to the second fastening part (3), receives the joint head (6) with pivoting movement, and has a neck opening (16), directed away from the fastening part (3) connected to it, for the passage of the neck part (17), characterized in that the enclosure part (4) has an installation opening (20) arranged laterally in relation to the neck opening (16).

2. Endoprosthesis according to Claim 1, characterized in that the enclosure part (4) is provided with a closing part (9) for holding the joint head (6) secure.

3. Endoprosthesis according to Claim 1 or 2, characterized in that the enclosure part (4) has a pot-shaped part (8) which is connected to the associated fastening part (3) and whose side wall (12) includes the installation opening (20).

4. Endoprosthesis according to one of Claims 1 to 3, characterized in that the closing part (9) is a ring which interacts with the wall (12) of the pot-shaped part (8) via a thread (14) or the like.

5. Endoprosthesis according to one of Claims 1 to 4, characterized in that a slide collar (18) is arranged between the joint head (6) and the enclosure part (4), said slide collar (8) being made of a material which promotes sliding, such as polyethylene.

6. Endoprosthesis according to Claim 5, characterized in that the slide collar is designed closed all around its side, and the width of the installation opening (20) corresponds at least to the external diameter of the slide collar (18).

7. Endoprosthesis according to Claim 5 or 6, characterized in that the slide collar (18) has an opening constriction (19) which can be passed through by the joint head (6) under elastic expansion of the slide collar, and in that the slide collar (18) is supported by the enclosure part (4) so as to obstruct expansion.

8. Endoprosthesis according to one of Claims 1 to 7, characterized in that the diameter of the neck opening (16) formed by the closing part (9) is greater than that of the joint head (6), but smaller than the external diameter of the slide collar (18) on the side with the opening.

9. Endoprosthesis according to one of Claims 1 to 8, characterized in that the joint head is spherical.

10. Endoprosthesis according to one of Claims 1 to 9, characterized in that the joint head (6) is not circular and is surrounded by an elastic sleeve.

## Revendications

1. Endoprothèse pour une articulation entre deux os qui ne peuvent être séparés l'un de l'autre, ou seulement difficilement, notamment pour l'articulation entre le sternum et la clavicule, comportant deux composants de prothèse (1, 2) qui présentent chacun un élément de fixation (3, 5) pour la fixation à l'os correspondant et qui forment une articulation, laquelle est constituée d'une tête d'articulation reliée au premier (5) des deux éléments de fixation, par un élément formant col (17), ainsi que d'un élément d'entourage (4) qui est relié au deuxième élément de fixation (3) et qui reçoit la tête d'articulation (6) mobile à pivotement, lequel élément d'entourage présente une ouverture de col (16), tournée à l'opposée de l'élément de fixation (3) qui lui est relié pour le passage de l'élément formant col (17), caractérisée en ce que l'élément d'entourage (4) présente une ouverture de montage (20) disposée latéralement par rapport à l'ouverture de col (16), pour l'introduction de la tête dans l'élément d'entourage.

2. Endoprothèse selon la revendication 1, caractérisée en ce que l'élément d'entourage (4) est pourvu d'un élément de fermeture (9), pour maintenir en place la tête d'articulation (6).

3. Endoprothèse selon la revendication 1, caractérisée en ce que l'élément d'entourage (4) présente une partie (8) en forme de pot, reliée à l'élément de fixation (3) correspondante, dont la paroi latérale (12) contient l'ouverture de montage (20).

4. Endoprothèse selon l'une des revendications 2 ou 3, caractérisée en ce que l'élément de fermeture (9) est un anneau coopérant avec la paroi (12) de la partie (8) en forme de pot, par un filetage (14) ou similaire.

5. Endoprothèse selon l'une des revendications 1 à 4, caractérisée en ce qu'une enveloppe de glissement (18) en matériau favorisant le glissement, tel que du polyéthylène, est disposée entre la tête d'articulation (6) et l'élément d'entourage (4).

6. Endoprothèse selon la revendication 5, caractérisée en ce que l'enveloppe de glissement est fermée tout autour sur les côtés et la largeur de l'ouverture de montage (20) correspond au moins au diamètre extérieur de l'enveloppe de glissement (18).

7. Endoprothèse selon la revendication 5 ou 6, caractérisée en ce que l'enveloppe de glissement (18) présente un rétrécissement d'ouverture (19), qui peut être traversé par la tête d'articulation (6), par dilatation élastique de l'enveloppe de glissement et en ce que l'enveloppe de glissement (18) est soutenue par l'élément d'entourage (4) de manière à empêcher la dilatation.

8. Endoprothèse selon l'une des revendications 1 à 7, caractérisée en ce que le diamètre de l'ouverture de col (16) formée par l'élément de fermeture (9), est supérieur à celui de la tête d'articulation (6), mais inférieur au diamètre extérieur côté ouverture de l'enveloppe de glissement (18).

9. Endoprothèse selon l'une des revendications 1 à 8, caractérisée en ce que la tête d'articulation est sphérique.

10. Endoprothèse selon l'une des revendications 1 à 8, caractérisée en ce que la tête d'articulation (6) est entourée de manière non circulaire et par une enveloppe élastique.
